# EUROPÄISCHE PATENTANMELDUNG

(11) **EP 2 399 511 A1**
(43) Veröffentlichungstag der Anmeldung: **28.12.2011**
(21) Anmeldenummer: 11171409.3
(22) Anmeldetag: 24.06.2011
(51) Int. Cl.: A61B 5/0295, A61B 5/107

(54) **Vorrichtung zur Messung einer Änderung des Blutvolumens in einer menschlichen Extremität**

(30) Priorität: 25.06.2010 DE 102010017606
(71) Anmelder: Gutmann MD GmbH, 82538 Geretsried (DE)
(72) Erfinder: Wolf, Jürgen, 82547 Eurasburg (DE)
(74) Vertreter: Wilhelms · Kilian & Partner Patentanwälte

(57) **Zusammenfassung**

Vorrichtung (1) zur Messung einer Änderung des Blutvolumens in einer menschlichen Extremität (10), umfassend ein Befestigungselement (4) zum Legen um die Extremität (10) mit einem ersten und einem zweiten Ende (6-1, 6-2), einen Inkrementalgeber (7) und eine Sensoreinheit (5), wobei das erste Ende (6-2) des Befestigungselements (4) an die Sensoreinheit (5) gekoppelt ist und das zweite Ende (6-1) des Befestigungselements (4) an den Inkrementalgeber (7) gekoppelt ist, und die Vorrichtung (1) dazu ausgebildet ist, eine Umfangsänderung der Extremität (10) basierend auf einer relativen Verschiebung des Inkrementalgebers (7) gegenüber der Sensoreinheit (5) zu ermitteln.

## Beschreibung

Die Erfindung betrifft eine Vorrichtung zur Messung einer Änderung des Blutvolumens in einer menschlichen Extremität, insbesondere im Rahmen einer Venenverschlussplethysmographie.

Venenerkrankungen der unteren Extremitäten sind sehr weit verbreitet und nehmen tendenziell immer mehr zu, Neuere epidemiologische Studien zeigen sehr deutlich eine zunehmende Krankheitshäufigkeit venöser Beinleiden und den damit verbundenen volkswirtschaftlichen Kosten. Deshalb kommt einer möglichst frühzeitigen Erkennung und Behandlung solcher Erkrankungen eine große Bedeutung zu. Nur bei rechtzeitiger Diagnose können irreversible Folgeschäden verhindert und ein chronischer Krankheitsverlauf verzögert bzw. gemildert werden.

Die Verfügbarkeit einer geeigneten und einfachen diagnostischen Methode ist eine wichtige Voraussetzung für eine schnelle Diagnose und effektive Beobachtung des Krankheitsverlaufs. Die Venenverschlussplethysmographie erfüllt diese Anforderungen und hat sich bereits seit vielen Jahren in der Thrombosediagnostik bewährt.

Bei der Venenverschlussplethysmographie wird der venöse Abfluss in der zu untersuchenden Extremität mittels einer Staumanschette ähnlich wie bei einer Blutdruckmessung zunächst blockiert und die Zunahme des Volumens und damit die venöse Kapazität infolge der arteriellen Einströmung des Blutes basierend auf einer Umfangsänderung der Extremität bestimmt. Nach Beendigung der Stauphase wird der Druck in der Staumanschette abgelassen, wodurch das aufgestaute Blut wieder abfließen kann und sich der Umfang der Extremität verringert. In Abhängigkeit von der aufgezeichneten zeitlichen Verringerung des Umfangs der Extremität kann ermittelt werden, ob die Venen durchgängig sind oder der Abstrom des Blutes beispielsweise durch einen Thrombus gestört ist.

Das Venensystem der unteren Extremität wird von den tiefen Leitvenen beherrscht. Diese führen 90% des arteriell eingepumpten Blutes dem Herzen wieder zu. Außerdem erfahren diese Venen auf Grund der Physiologie des Menschen die stärksten Belastungen. Aus diesen Gründen haben die Untersuchungen an unteren Extremitäten eine hohe therapeutische Bedeutung.

Zur Erfassung des Blutvolumens am Unterschenkel sind verschiedene Methoden bekannt.

Aus der DE 1 566 075 A ist ein Verfahren zur Messung des Bluteinstromvolumens in Gefäßbereiche bekannt, welche auf der Dehnungsmessstreifen-Methode von WHITNEY basiert. Dabei wird ein elastischer Gummischlauch verwendet, der mit Quecksilber gefüllt ist, und um die zu untersuchende Extremität gelegt wird. Da sich der elektrische Widerstand der eingeschlossenen Quecksilkbersäule proportional zu ihrer Länge ändert, kann auf die Umfangsänderung der Extremität geschlossen werden. Zur effektiveren Verteilung des Drucks wird der Gummischlauch durch Gleitglieder von der Haut getrennt, Nachteilig an dieser Vorrichtung ist, dass das Quecksilber im Falle eines Austritts aus dem Gummischlauch eine hohe Umwelt- und Gesundheitsgefährdung darstellt.

Aus der DE 198 57 098 A1 ist eine Vorrichtung zur Bestimmung eines Umfangs eines Körpers bekannt, welche einen Draht mit einem bekannten Längenabschnitt aufweist, der um den Körper gelegt und mittels einer Antriebseinrichtung gespannt wird. Die Antriebseinrichtung ist an einen Wegmesser zur Messung des Weges, den der Draht während des Spannens zurücklegt, gekoppelt. Sobald eine von dem Draht auf einen Lagesensor ausgeübte Zugkraft einen vorbestimmten Wert überschreitet, wird ein Signal erzeugt und die Antriebseinrichtung gestoppt. Der Umfang des Körpers kann dann aus dem bekannten Längenabschnitt des Drahtes, dem während des Spannens zurückgelegten Weg des Drahtes und einem vorrichtungsabhängigen Geometriefaktor bestimmt werden. Nachteilig an dieser Vorrichtung ist, dass sie aufgrund der Verwendung einer Antriebseinrichtung einen relativ hohen Raumbedarf aufweist.

Es ist eine Aufgabe der vorliegenden Erfindung, eine Vorrichtung zur Messung einer Änderung des Blutvolumens in einer menschlichen Extremität bereitzustellen, welche keine Gesundheitsgefährdenden Stoffe enthält und einen geringen Raumbedarf aufweist.

Diese Aufgabe wird gelöst durch eine Vorrichtung zur Messung einer Änderung des Blutvolumens in einer menschlichen Extremität, welche ein Befestigungselement zum Legen um die Extremität mit einem ersten und einem zweiten Ende, einen Inkrementalgeber und eine Sensoreinheit umfasst, wobei das erste Ende des Befestigungselements an die Sensoreinheit gekoppelt ist und das zweite Ende des Befestigungselements an den Inkrementalgeber gekoppelt ist, und die Vorrichtung dazu ausgebildet ist, eine Umfangsänderung der Extremität basierend auf einer relativen Verschiebung des Inkrementalgebers gegenüber der Sensoreinheit zu ermitteln.

Der Inkrementalgeber kann eine Vielzahl von in gleichmäßigen Abständen entlang einer Bewegungsrichtung des Inkrementalgebers angeordnete optische oder magnetische Markierungen umfassen, wobei die Sensoreinheit die Markierungen abtastet.

Gemäß einer Ausführungsform umfasst die Vorrichtung eine Zugvorrichtung, beispielsweise eine Rückstellfeder, welche auf den Inkrementalgeber eine Zugkraft entlang einer Bewegungsrichtung des Inkrementalgebers ausübt, in welche sich der Inkrementalgeber im Falle einer Umfangsverringerung der Extremität bewegt.

Durch das Vorsehen der Zugvorrichtung wird das Befestigungselement insbesondere bei der Messung gespannt, wodurch eine verlässliche Messung der Umfangsänderung ermöglicht wird.

Bevorzugt umfasst die Vorrichtung ein Gehäuse und eine in dem Gehäuse integrierte Auswerteeinheit, welche basierend auf von der Sensoreinheit zugeführten Ausgangssignalen die Umfangsänderung der Extremität ermittelt. Durch die Integration des Inkrementsigebers, der Sensoreinheit und der Auswerteeinheit in das Gehäuse der Vorrichtung können potentielle Störquellen bei einer Signalübertragung reduziert werden.

Gemäß einer Weiterbildung umfasst die Vorrichtung einen Schlitten, der in dem Gehäuse gleitend beweglich angeordnet ist und an welchem der Inkrementalgeber und das zweite Ende des Befestigungselements befestigt sind. In diesem Fall ist ein Ende der Rückstellfeder bevorzugt an dem Schlitten befestigt und ein anderes Ende der Rückstellfeder an das Gehäuse gekoppelt.

Gemäß einer Ausführungsform weist das Befestigungselement mindestens zwei Bänder und eine Längenverstelleinheit zur Einstellung einer Gesamtlänge des Befestigungselements durch Verschiebung eines Stellriegels auf. Eine Information über die Verschiebung des Stellriegels wird der Auswerteeinheit zugeführt, welche basierend auf der Verschiebung des Stellriegels, einer Länge der mindestens zwei Bänder und den von der Sensoreinheit zugeführten Ausgangssignalen eine relative Umfangsänderung der Extremität ermittelt. Die relative Umfangsänderung ist dabei durch den Quotienten aus der ermittelten Umfangsänderung und der Gesamtlänge des Befestigungselements definiert. Basierend auf der relativen Umfangsänderung kann dann mittels der Auswerteeinheit die Änderung des Blutvolumens in der Extremität berechnet werden.

Die Vorrichtung kann ferner eine in dem Gehäuse integrierte Anzeigeeinheit aufweisen, welche beispielsweise mehrere lichtemittierende Dioden (LEDs) umfasst, die Licht unterschiedlicher Farbe emittieren, und welche eine Information über eine Position des Inkrementalgebers anzeigt.

Durch das Vorsehen der Anzeigeeinheit wird der Benutzer beim Anlegen des Befestigungselements und insbesondere bei der Einstellung der Längenverstelleinheit unterstützt. So kann beispielsweise eine rote LED aufleuchten, wenn das Befestigungselement so locker oder so eng um die Extremität gelegt ist, dass sich der Inkrementalgeber an einer Position befindet, die außerhalb eines Erfassungsbereichs der Sensoreinheit liegt, und eine Messung somit nicht möglich ist.

Wenn hingegen eine Messung auf Grund der Position des Inkrementalgebers möglich ist, kann dies dem Benutzer durch Aufleuchten einer grünen oder gelben LED angezeigt werden.

Die Vorrichtung kann ferner mindestens ein Gleitelement umfassen, welches zur Beabstandung der mindestens zwei Bänder des Befestigungselements von der Extremität vorgesehen ist, und welches entlang einer Bewegungsrichtung der mindestens zwei Bänder verlaufende Rippen aufweist. Durch das Vorsehen der Rippen wird vorteilhaft die Reibfläche zwischen den Bändern und dem Gleitelement reduziert.

An die Auswerteeinheit kann eine Datenverarbeitungseinhert zur Speicherung, Darstellung und Weiterverarbeitung der von der Auswerteeinheit ermittelten Daten gekoppelt sein.

Gemäß einer Ausführungsform umfasst der Inkrementalgeber eine Vielzahl von magnetischen Abschnitten, welche nacheinander entlang der Bewegungsrichtung des Inkrementalgebers angeordnet sind und alternierend unterschiedliche magnetische Polarisierungsrichtungen aufweisen. In diesem Fall weist die Sensoreinheit bevorzugt eine Vielzahl von linearen Hallsensoren auf.

Die einzelnen Abschnitte der Vielzahl von magnetischen Abschnitten weisen bevorzugt eine entlang der Bewegungsrichtung des Inkrementalgebers gemessene Länge von 2 mm auf, und die Sensoreinheit umfasst bevorzugt 32 Hallsensoren, wobei Hallsensoren jeweiliger Paare von Hallsensoren versetzt angeordnet sind, um eine Bewegungsrichtung des Inkrementalgebers bestimmen zu können. Mit dieser Konfiguration und einer geeigneten Verarbeitung der Ausgangssignale der Hallsensoren durch die Auswerteeinheit wird eine Auflösung für die Wegmessung erreicht, die weniger als 1 µm beträgt.

Gemäß einer anderen Ausführungsform ist der Inkrementalgeber als Streifen aus einem lichtdurchlässigen Medium ausgebildet, auf dem lichtundurchlässige Markierungen aufgebracht sind, oder als Streifen, welcher aus alternierend angeordneten reflektiven und nicht-reflektiven Markierungen besteht. In diesem Fall weist die Sensoreinheit zumindest eine Leuchtdiode und eine Photodiode auf.

Im Folgenden wird anhand der zugehörigen Zeichnungen ein Ausführungsbeispiel einer Vorrichtung zur Messung einer Änderung des Blutvolumens in einer menschlichen Extremität näher beschrieben.

Es zeigen:
Fig. 1 eine Prinzipskizze einer Vorrichtung zur Messung einer Änderung des Blutvolumens in einer menschlichen Extremität gemäß einer Ausführungsform,
Fig. 2 ein Gleitelement der in Fig. 1 gezeigten Vorrichtung,
Fig. 3 eine schematische Vorderansicht einer Vorrichtung zur Messung einer Änderung des Blutvolumens in einer menschlichen Extremität gemäß einer Ausführungsform,
Fig. 4 eine Querschnittsansicht der in Fig. 3 dargestellten Vorrichtung entlang der in Fig. 3 gezeigten Schnittlinie FF,
Fig. 5 eine Detailansicht des in Fig. 4 dargestellten Teilbereichs H,
Fig. 6 eine Querschnittsansicht der in Fig. 3 dargestellten Vorrichtung entlang der in Fig. 3 gezeigten Schnittlinie G-G,
Fig. 7 eine Querschnittsansicht einer Längenverstelleinheit zur Anpassung der Länge eines Befestigungselements der Vorrichtung an eine Extremität,
Fig. 8 eine schematische perspektivische Ansicht eines Teils der in Fig. 7 gezeigten Längenverstelleinheit, und
Fig. 9 eine schematische Ansicht eines Teils der in Fig. 7 gezeigten Längenverstelleinheit.

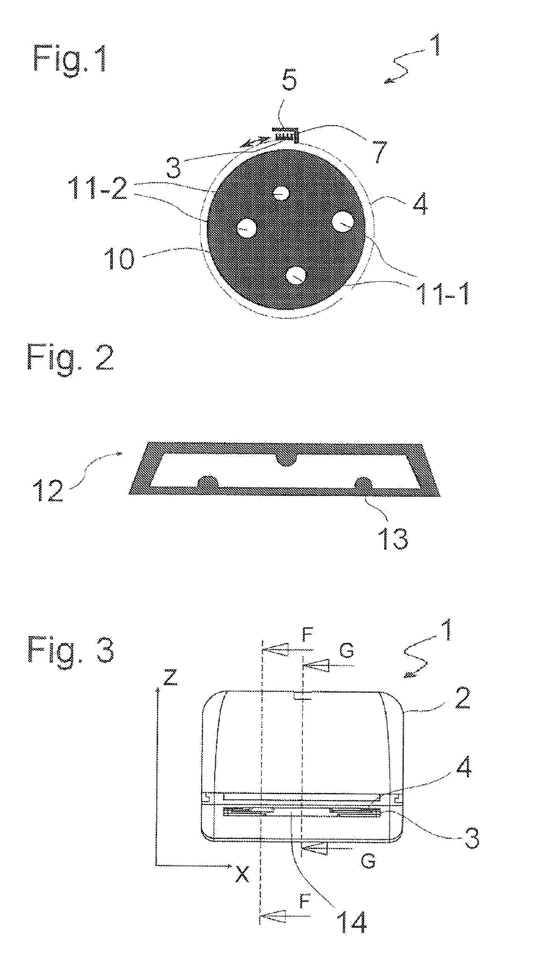

Fig. 1 zeigt eine Prinzipskizze einer Vorrichtung zur Messung einer Änderung des Blutvolumens in einer menschlichen Extremität gemäß einer Ausführungsform. Die Vorrichtung 1 umfasst ein Befestigungselement 4, welches beispielsweise zwei flexible Bänder und eine die beiden Bänder verbindende Längenverstelleinheit aufweist, und welches um eine Extremität 10 eines menschlichen Körpers, wie etwa einen Arm, ein Bein oder einen Finger gelegt ist.

Ein Ende des Befestigungselements 4 ist fest mit einer Sensoreinheit 5 verbunden, und das andere Ende des Befestigungselements 4 ist mit einem Schlitten 3 verbunden, auf welchem ein Inkrementalgeber 7 angeordnet ist.

Bei einer Aufstauung des Blutes mittels einer Staumanschette (nicht gezeigt), welche weiter entfernt vom Herzen des Menschen als das Befestigungselement 4 angebracht wird, kann das über die Arterien 11-1 zugeführte Blut nicht über die Venen 11-2 abfließen. Aus diesem Grund nimmt der Umfang der Extremität 10 zu, wodurch der Schlitten 3 in Umfangsrichtung de Extremität bewegt wird. Die Länge des Weges, die der Schlitten 3 zurücklegt, wird mittels des Inkrementalgebers 7, der Sensoreinheit 5 und einer nicht gezeigten Auswerteeinheit bestimmt. Basierend auf der bestimmten Weglänge des Schlittens 3 kann dann die Umfangsänderung der Extremität 10 und die Änderung des Blutvolumens beispielsweise mittels eines an die Auswerteeinheit angeschlossenen PCs bestimmt werden.

Der Inkrementalgeber 7 kann eine Vielzahl von in gleichmäßigen Abständen entlang einer Bewegungsrichtung des Schlittens 3 angeordnete optische oder magnetische Markierungen oder Codierungen umfassen.

Weiterhin kann der Inkrementalgeber 7 als ein Element ausgebildet sein, welches entlang einer Bewegungsrichtung des Inkrementalgebers 7 beabstandet angeordnete Zähne aufweist. In diesem Fall ist die Sensoreinheit 5 als ein Sensor ausgebildet, welcher bei einer Bewegung des Inkrementalgebers 7 in Folge einer Änderung eines induktiven Widerstands ein Signal erzeugt. Bei entsprechender Form der Zähne wird insbesondere ein sinusähnlicher Spannungsverlauf erzeugt, aus welchem mittels einer Auswerteeinheit eine Umfangsänderung der Extremität ermittelt wird,

Als optischer Inkrementalgeber 7 kann beispielsweise ein Streifen verwendet werden, welcher ein lichtdurchlässiges Medium umfasst, auf dem lichtundurchlässige Markierungen aufgebracht sind. Weiterhin kann als optischer Inkrementalgeber 7 auch ein Streifen verwendet werden, welcher aus alternierend angeordneten reflektiven und nicht-reflektiven Markierungen besteht.

Im Falle eines optischen Inkrementalgebers 7 besteht die Sensoreinheit 5 aus einer oder mehreren Leuchtdioden, denen jeweils zwei zueinander versetzt angeordnete Photodioden zugeordnet sind. Bei Verwendung eines optischen Inkrementalgebers 7 mit einem Streifen, welcher ein lichtdurchlässiges Medium umfasst, auf dem lichtundurchlässige Markierungen aufgebracht sind, sind die Leuchtdiode und die Photodioden auf unterschiedlichen Seiten bezüglich des Befestigungselements 4 angeordnet. Die Photodioden können beispielsweise unterhalb des Schlittens 3 vorgesehen sein, welcher in diesem Fall ebenfalls aus einem lichtdurchlässigen Material besteht. Der Strahl der Leuchtdiode wird entsprechend den Markierungen auf dem Streifen unterbrochen oder durchgelassen.

Bei Verwendung eines optischen Inkrementalgebers 7 mit alternierend angeordneten reflektiven und nicht-reflektiven Markierungen sind die Leuchtdiode und die Photodioden auf derselben Seite bezüglich des Befestigungselements 4 angeordnet.

Wird der optische Inkrementalgeber 7 in Folge einer Umfangsänderung der Extremität 10 bewegt, so erzeugt die Photodiode durch die Änderung der Lichtdurchlässigkeit bzw. der Reflexion eine Anzahl von elektrischen Pulsen, die der Anzahl der Markierungen auf dem Inkrementalgeber 7 entspricht. Aus der Anzahl der Pulse kann mittels einer Auswerteeinheit auf eine Umfangsänderung und damit auf eine Änderung des Blutvolumens in der Extremität 10 geschlossen werden,

Als magnetischer Inkrementalgeber 7 kann ein Magnetgeber verwendet werden, welcher eine Vielzahl von nacheinander entlang einer Bewegungsrichtung des Inkrementalgebers 7 angeordnete magnetische Abschnitte umfasst, die alternierend unterschiedliche Polarisierungsrichtungen aufweisen. In diesem Fall ist die Sensoreinheit 5 bevorzugt als ein Array aus einer Vielzahl von linearen Hallsensoren ausgebildet. Um die Bewegungsrichtung des Inkrementalgebers 7 bestimmen zu können, sind jeweils Paare der Vielzahl von linearen Hallsensoren versetzt angeordnet. Die linearen Hallsensoren liefern jeweils eine Analogspannung, die proportional zu dem durch die magnetischen Abschnitte des Magnetgebers normal auf den jeweiligen Sensor wirkenden Magnetfeld ist. Somit erzeugt ein bewegter Magnetgeber ein jeweiliges Sinussignal am Ausgang eines entsprechenden linearen Hallsensors. Durch geeignete Verschaltung der am Ausgang der linearen Hallsensoren anliegenden Signale wird mittels der Auswerteeinheit die Länge des Weges, um die sich der Inkrementalgeber 7 relativ zu der Sensoreinheit 5 bewegt, die Bewegungsrichtung und somit die positive oder negative Umfangsänderung der Extremität bestimmt,

Um ein optimales Gleitverhalten zu realisieren, wird das Befestigungselement 4 über einzelne nicht gezeigte Gleitglieder geführt, die eine reibungsminimierte Innengeometrie aufweisen.

Fig, 2 zeigt ein Gleitelement der Vorrichtung zur Messung einer Änderung des Blutvolumens in einer menschlichen Extremität gemäß einer Ausführungsform. Das Gleitelement 12 weist beispielsweise Ösen (nicht gezeigt in Fig. 2) auf, durch welche die Bänder des Befestigungselements 4 gefädelt sind, und dient dazu, das Band des Befestigungselements 4 von der Haut der Extremität 10 zu beabstanden. Am Gleitelement 12 sind entlang einer Bewegungsrichtung des Bandes verlaufende Rippen 13 angeordnet, welche die Reibfläche zwischen dem Band und dem Gleitelement 12 reduzieren. Die Bänder des Befestigungselements 4 sind zwar biegsam, damit sie um die Extremität 10 gelegt werden können. Jedoch dürfen sie nicht oder nur geringfügig dehnbar sein, da ansonsten in Folge der Dehnung der Bänder des Befestigungselements 4 eine zuverlässige Messung der Umfangsänderung nicht möglich wäre. In einer bevorzugten Ausführungsform ist das Band des Befestigungselements 4 als Flachbandkabel mit Kupferadern ausgebildet.

Fig. 3 zeigt eine schematische Vorderansicht einer Vorrichtung zur Messung einer Änderung des Blutvolumens in einer menschlichen Extremität gemäß einer Ausführungsform. Die Vorrichtung 1 weist ein Gehäuse 2 auf, in welchem ein Schlitz 14 vorgesehen ist, durch den ein Ende eines Bandes des Befestigungselements 4, das mit Abschnitten des Schlittens 3 verbunden ist, durchgeführt ist.

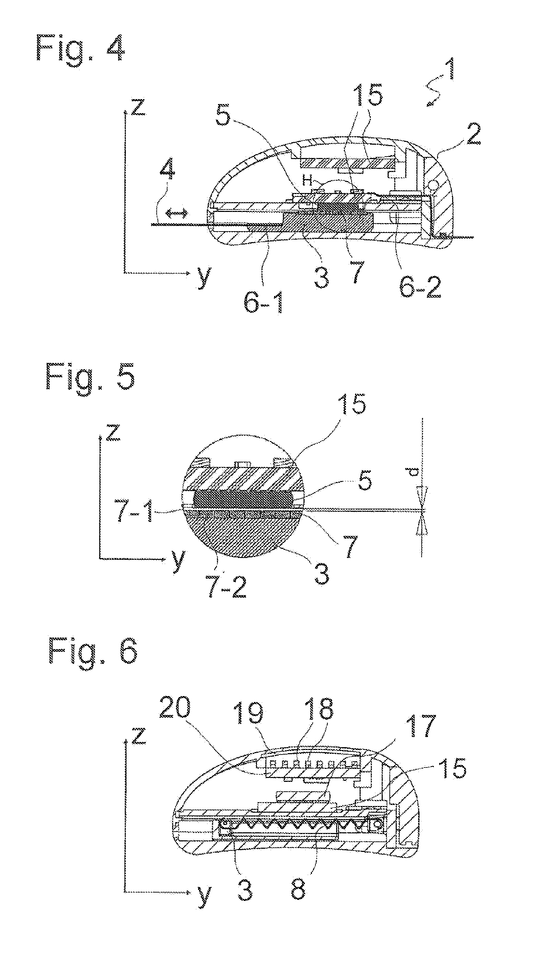

Fig. 4 zeigt eine Querschnittsansicht der in Fig. 3 gezeigten Vorrichtung entlang der in Fig. 3 gezeigten Schnittlinie F-F. Die Unterseite des Gehäuses 2 ist mit einer Wölbung versehen, um eine verbesserte Auflage auf einer Extremität eines menschlichen Körpers zu ermöglichen. Innerhalb des Gehäuses 2 ist der Schlitten 3, der mit einem Ende 6-1 eines Bandes des Befestigungselements 4 verbunden ist, entlang einer Richtung Y gleitend beweglich angeordnet. Auf dem Schlitten 3 ist ein als Magnetgeber ausgebildeter Inkrementalgeber 7 angeordnet. Oberhalb des Schlittens 3 ist eine als ein Array aus einer Vielzahl von linearen Hallsensoren ausgebildete Sensoreinheit 5 vorgesehen, die mit dem Ende 6-2 eines anderen Bandes des Befestigungselements 4 fest verbunden ist.

Die Sensoreinheit 5 ist an eine Auswerteinhest 15 gekoppelt, welcher die Ausgangssignale der einzelnen Hallsensoren mittels nicht dargestellter elektrischer Leitungen zugeführt werden. Die Auswerteeinheit 15 ermittelt dann basierend auf den zugeführten Ausgangssignalen der einzelnen linearen Hallsensoren eine relative Verschiebung des Inkrementalgebers 7 gegenüber der Sensoreinheit 5 und somit eine Umfangsänderung der Extremität.

Fig. 5 zeigt eine Detailansicht des in Fig. 4 dargestellten Teilbereichs H. Der Magnetgeber 7 weist eine Vielzahl von magnetischen Abschnitten 7-1, 7-2 auf, welche abwechselnd entlang der Y-Richtung angeordnet sind und alternierend unterschiedliche magnetische Polarisierungsrichtungen in der Z-Richtung aufweisen. Der Schlitten 3 ist derart angeordnet und gelagert, dass ein in Z-Richtung gemessener Abstand d von gegenüberliegenden Oberflächen des Inkrementalgebers 7 und der Sensoreinheit 5 etwa 0,15 ± 0,5 mm beträgt. Die einzelnen Abschnitte 7-1, 7-2 des Magnetgebers 7 weisen in Y-Richtung gemessen eine Länge von 2 mm auf. Das Hallsensorarray besteht aus 32 linearen Hallsensoren, wodurch durch die Verarbeitung der Ausgangssignale der einzelnen linearen Hallsensoren mittels der Auswerteeinheit 15 eine Auflösung für die Wegmessung des Schlittens 3 erreicht wird, die kleiner als 1 µm ist.

Bei der Vorrichtung 1 findet die Erfassung der Umfangsänderung der Extremität somit vorteilhaft direkt am Gehäuse 2 statt, wodurch potentielle Störquellen bei einer Signalübertragung reduziert werden.

Fig. 6 zeigt eine Querschnittsansicht der in Fig. 3 gezeigten Vorrichtung 1 entlang der in Fig. 3 gezeigten Schnittlinie G-G. Auf der Auswerteeinheit 15 ist ein Mikrocontroller 17 angeordnet, weicher die Vorrichtung 1 steuert. Unterhalb eines transparenten Fensters 19 des Gehäuses 2 sind mehrere lichtemittierende Dioden (LEDs) 18 vorgesehen, welche in Abhängigkeit von der Position des Schlittens 3, die von der Auswerteeinheit 15 ermittelt wird, von einem Steuermodul 20 angesteuert und zum Leuchten gebracht werden. Dabei sind die in der Fig. 6 am weitesten links und die am weitesten rechts befindlichen LEDs 18 als rote LEDs, die jeweils benachbarten LEDs als gelbe LEDs, und die vier mittleren LEDs als grüne LEDs ausgebildet.

Auf den Schlitten 3 wird mittels einer Rückstellfeder 8 eine Zugkraft entlang der Y-Richtung ausgeübt, wodurch das Befestigungselement 4 bei der Messung gespannt wird. Dabei ist die Rückstellkraft der Rückstellfeder 8 derart gewählt, dass sie kleiner ist, als die durch den Venendruck von etwa 12 mmHg auf das Befestigungselement 4 ausgeübte Zugkraft.

Wenn in negativer Y-Richtung durch das Befestigungselement 4 keine Kraft auf den Schlitten 3 ausgeübt wird, das Befestigungselement 4 also beispielsweise zu locker um die Extremität gelegt ist, wird der Schlitten 3 mittels der Rückstellfeder 8 in einer Anschlagposition auf der rechten Seite gehalten, die außerhalb des Messbereichs der Vorrichtung 1 liegt. Dies wird dem Benutzer durch Aufleuchten der in der Fig. 6 am weitesten rechts befindlichen roten LED 18 angezeigt.

Ist hingegen das Befestigungselement 4 zu eng angelegt, d. h. so stark gespannt, dass die resultierende Zugkraft die Rückstellkraft der Rückstellfeder 8 überschreitet, so befindet sich der Schlitten 3 in einer Anschlagposition auf der linken Seite, weiche außerhalb des Messbereichs liegt. Dies wird dem Benutzer durch Aufleuchten der in der Fig. 6 am weitesten links befindlichen roten LED 18 angezeigt.

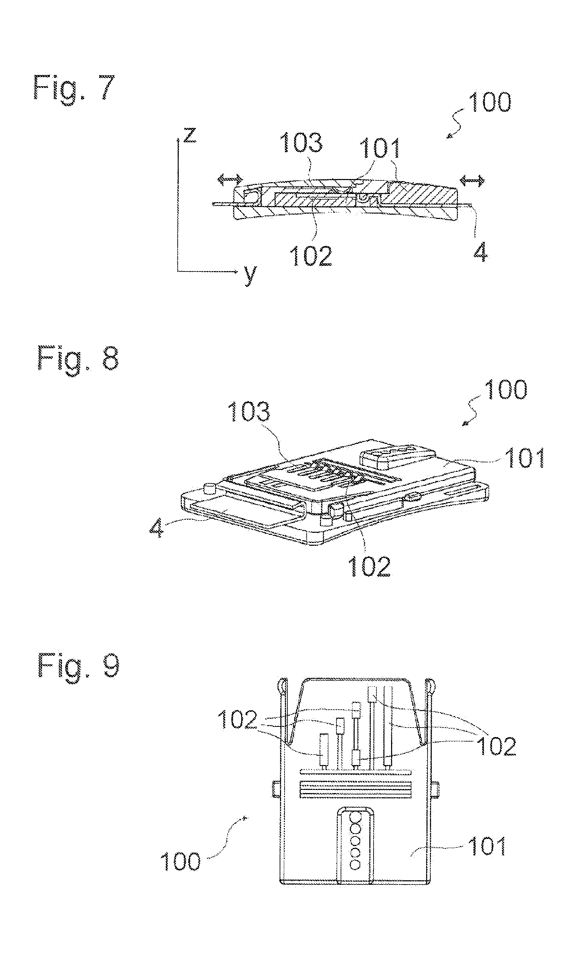

Der Benutzer wird somit durch die Anzeige der roten LEDs 18 darauf hingewiesen, dass keine Messung möglich ist, und er die Länge des Befestigungselements 4 mit der mit Bezug auf die Fig. 7 bis 9 beschriebenen Längenverstelleinheit anpassen muss.

Befindet sich der Schlitten 3 in einer mittleren Position, so wird dem Benutzer durch Aufleuchten einer der grünen oder einer der gelben LEDs 18 angezeigt, dass er eine Messung starten kann.

Zur Speicherung und weiteren Auswertung der Daten umfasst die Vorrichtung 1 ferner eine nicht dargestellte Datenverarbeitungseinheit wie etwa einen PC oder Laptop mit entsprechender Software, welche über am Gehäuse 2 vorgesehene Anschlüsse (nicht gezeigt) angeschlossen wird. Dabei kann die Auswertung der Daten beispielsweise die Darstellung einer bekannten Volumenkurve auf einem Bildschirm umfassen.

Auf Grund der Verwendung eines Inkrementalgebers 7 und einer entsprechend angepassten Sensoreinheit 5 weist das Gehäuse 2 einen im Vergleich zu bekannten Vorrichtungen geringeren Raumbedarf auf. In der in den Fig. 3 bis 6 gezeigten Ausführungsform weist das Gehäuse 2 beispielsweise lediglich eine Grundfläche von 1,5 x 2 cm² auf.

Fig. 7 zeigt eine Querschnittsansicht einer Längenverstelleinheit 100 des Befestigungselements 4, welche zur Anpassung der Länge des Befestigungselements 4 an eine Extremität, deren Umfangsänderung in Folge einer Änderung des Blutvolumens gemessen werden soll, dient. Die Fig. 8 und 9 zeigen schematische Ansichten von Teilen der in Fig. 7 gezeigten Längenverstelleinheit.

Die Längenverstelleinheit 100 umfasst einen Stellriegel 101, der mit einem Ende eines Bandes des Befestigungselements 4, das mit der Sensoreinheit 5 verbunden ist, fest verbunden ist und entlang der in der Fig. 7 gezeigten Y-Richtung verschiebbar angeordnet ist. Ein Ende eines anderen Bandes des Befestigungselements 4, das mit dem Schlitten 3 verbunden ist, ist fest mit dem Gehäuse der Längenverstelleinheit 100 verbunden.

In dem Sltellriegel 101 sind, wie in Fig. 9 gezeigt, mehrere Rasteinrichtungen 102 an unterschiedlichen Position vorgesehen, welche elektrische Kontakte aufweisen. Mittels einer darüber angeordneten Abnehmerfeder 103, welche, wie in Fig. 8 gezeigt, mehrere Arme aufweist, die in Abhängigkeit von der Y-Position des Stellriegels 101 in eine oder mehrere der Rasteinrichtungen 102 eingreifen, kann die Verschiebung des Stellriegels 101 zur Einstellung einer Gesamtlänge des Befestigungselements 4 entlang der Y-Richtung ermittelt werden. Die ermittelte Verschiebung des Stellriegels 101 wird über in einem Band des Befestigungselements 4 vorgesehene elektrische Leitungen an die Auswerteeinheit 15 übertragen. Die Auswerteeinheit 15 ermittelt dann basierend auf der Verschiebung und vorbestimmten Längen der beiden Bänder des Befestigungselements 4 die vor Beginn einer Messung eingestellte Gesamtlänge des Befestigungselements 4 und die relative Umfangsänderung der Extremität, d. h. den Quotienten aus der ermittelten Umfangsänderung und der Gesamtlänge des Befestigungselements 4. Aus der relativen Umfangsänderung kann dann die Änderung des Blutvolumens mittels der Auswerteeinheit 15 bestimmt werden,

In dem in den Fig. 7 bis 9 dargestellten Ausführungsbeispiel einer Langenverstelleinheit 100 kann die Länge des Befestigungselements 4 im Bereich von 0 bis 10 mm Stufenweise verändert werden. Eine entsprechende Codierung der in Fig. 9 dargestellten elektrischen Kontakte der Rasteinrichtungen 102 ist in der Tabelle 1 dargestellt.

**Tabelle 1**

| Kontakt | 0 mm | 3 mm | 4 mm | 6 mm | 8 mm | 10 mm |
|---|---|---|---|---|---|---|
| 1 | Dauerkontakt | | | | | |
| 2 | | | | | X | |
| 3 | X | | | X | | |
| 4 | | | X | | | |
| 5 | X | X | | | | |

Bei der Messung wird zunächst das Befestigungselement 4 zusammen mit dem Gehäuse 2 über die Extremität gestreift, deren Umfangsänderung in Folge einer Änderung des Blutvolumens gemessen werden soll, und das Gehäuse 2 mit einer Datenverarbeitungseinheit wie etwa einem PC oder einem Laptop verbunden. Dann wird mittels der Längenverstelleinheit 100 die Länge des Befestigungselements 4 derart eingestellt, dass der Schlitten 3 sich im Gehäuse 2 in einer mittleren Position befindet. Dabei wird der Benutzer von einer Anzeige der LEDs 18 unterstützt. Nach dieser Phase wird ein Systemtest durchgeführt, bei dem alle Elemente des Messsystems auf eine korrekte Funktion hin überprüft werden.

Danach werden mittels einer Staumanschette je nach Verfahren gegebenenfalls mehrfach unterschiedliche Drücke auf die Extremität gegeben, wodurch das Blut in der Extremität gestaut wird. Dabei wird der Umfang der Extremität mittels der Vorrichtung 1 kontinuierlich überwacht. Die Daten hierzu werden an die Datenverarbeitungseinheit weitergegeben und dort gespeichert, dargestellt und gegebenenfalls automatisch mittels einer entsprechenden Software vorausgewertet.

## Patentansprüche

1. Vorrichtung (1) zur Messung einer Änderung des Blutvolumens in einer menschlichen Extremität (10), umfassend:
ein Befestigungselement (4) zum Legen um die Extremität (10) mit einem ersten und einem zweiten Ende (6-1, 6-2),
einen Inkrementalgeber (7) und
eine Sensoreinheit (5), wobei
das erste Ende (6-2) des Befestigungselements (4) an die Sensoreinheit (5) gekoppelt ist und das zweite Ende (6-1) des Befestigungselements (4) an den Inkrementalgeber (7) gekoppelt ist, und die Vorrichtung (1) dazu ausgebildet ist, eine Umfangsänderung der Extremität (10) basierend auf einer relativen Verschiebung des Inkrementalgebers (7) gegenüber der Sensoreinheit (5) zu ermitteln.

2. Vorrichtung (1) nach Anspruch 1, wobei der Inkrementalgeber (7) eine Vielzahl von in gleichmäßigen Abständen entlang einer Bewegungsrichtung des Inkrementalgebers (7) angeordnete optische oder magnetische Markierungen (7-1, 7-2) umfasst, und die Sensoreinheit (5) die Markierungen abtastet.

3. Vorrichtung (1) nach Anspruch 1 oder 2, ferner umfassend eine Zugvorrichtung (8), welche auf den Inkrementalgeber (7) eine Zugkraft entlang einer Bewegungsrichtung des Inkrementalgebers (7) ausübt, in welche sich der Inkrementalgeber (7) im Falle einer Umfangsverringerung der Extremität (10) bewegt.

4. Vorrichtung (1) nach einem der Ansprüche 1 bis 3, ferner umfassend ein Gehäuse (2) und eine in dem Gehäuse (2) integrierte Auswerteeinheit (15), weiche basierend auf von der Sensoreinheit (5) zugeführten Ausgangssignalen die Umfangsänderung der Extremität (10) ermittelt.

5. Vorrichtung (1) nach Anspruch 4, ferner umfassend einen Schlitten (3), der in dem Gehäuse (2) gleitend beweglich angeordnet ist, und an welchem der Inkrementalgeber (7) und das zweite Ende (6-1) des Befestigungselements (4) befestigt sind.

6. Vorrichtung (1) nach einem der Ansprüche 4 oder 5, bei der das Befestigungselement (4) mindestens zwei Bänder und eine Längenverstelleinheit (100) zur Einstellung einer Gesamtlänge des Befestigungselements (4) durch Verschiebung eines Stellriegels (101) aufweist, und eine Information über die Verschiebung des Stellriegels (101) der Auswerteeinheit (15) zugeführt wird, welche basierend auf der Verschiebung des Stellriegels (101), einer Länge der mindestens zwei Bänder und den von der Sensoreinheit (5) zugeführten Ausgangssignalen eine relative Umfangsänderung der Extremität (10) ermittelt.

7. Vorrichtung (1) nach einem der Ansprüche 4 bis 6, ferner umfassend eine in dem Gehäuse (2) integrierte Anzeigeeinheit (18), welche eine Information über eine Position des Inkrementalgebers (7) anzeigt.

8. Vorrichtung (1) nach Anspruch 6 oder 7, ferner umfassend mindestens ein Gleitelement (12), welches zur Beabstandung der mindestens zwei Bänder des Befestigungselements (4) von der Extremität (10) vorgesehen ist, und welches entlang einer Bewegungsrichtung der mindestens zwei Bänder verlaufende Rippen (13) aufweist.

9. Vorrichtung (1) nach einem der Ansprüche 4 bis 8, ferner umfassend eine an die Auswerteeinheit (15) gekoppelte Datenverarbeitungseinheit zur Speicherung, Darstellung und Weiterverarbeitung der von der Auswerteeinheit (15) ermittelten Daten.

10. Vorrichtung (1) nach einem der Ansprüche 2 bis 9, bei der der Inkrementalgeber (7) eine Vielzahl von magnetischen Abschnitten (7-1, 7-2) umfasst, welche nacheinander entlang der Bewegungsrichtung des Inkrementalgebers (7) angeordnet sind und alternierend unterschiedliche magnetische Polarisierungsrichtungen aufweisen, und die Sensoreinheit (5) eine Vielzahl von linearen Hallsensoren umfasst.

11. Vorrichtung (1) nach einem der Ansprüche 2 bis 9, bei der der Inkrementalgeber (7) als Streifen aus einem lichtdurchlässigen Medium ausgebildet ist, auf dem lichtundurchlässige Markierungen aufgebracht sind, oder als Streifen, welcher aus alternierend angeordneten reflektiven und nicht-reflektiven Markierungen besteht, und die Sensoreinheit (5) zumindest eine Leuchtdiode und eine Photodiode umfasst.
